# FASCICULE DE BREVET EUROPEEN

(11) **EP 1 403 278 B1**
(45) Date de publication et mention de la délivrance du brevet: **08.06.2005**
(21) Numéro de dépôt: 03292404.5
(22) Date de dépôt: 30.09.2003
(51) Int. Cl.: C07K 5/06, C07C 251/08, C07C 229/30, C07C 227/32, C07K 5/02

(54) **Procédé de synthèse de la N-((S)-1-carbéthoxybutyl)-(S)-alanine et application à la synthèse du perindopril**
Verfahren zur Herstellung von N-((S)-1-(Ethoxycarbonyl)butyl)-(S)-alanin und Verwendung in der Synthese von Perindopril
Process for the synthesis of N-((S)-1-(ethoxycarbonyl)butyl)-(S)-alanine and use in the synthese of perindopril

(43) Date de publication de la demande: 31.03.2004
(73) Titulaire: LES LABORATOIRES SERVIER, 92415 Courbevoie Cédex (FR)
(72) Inventeur: Breard, Fabienne, 76140 Petit Quevilly (FR); LeCouve, Jean-Pierre, 76600 Le Havre (FR)

(56) Documents cités:
- WO-A-01/56353
- WO-A-01/56972
- BENNION C ET AL: "Design, synthesis, and physicochemical properties of a novel, conformationally restricted 2,3-dihydro-1,3,4-thiadiazole-containing angiotensin converting enzyme inhibitor which is preferentially eliminated by the biliary route in rats" JOURNAL OF MEDICINAL CHEMISTRY, AMERICAN CHEMICAL SOCIETY. WASHINGTON, US, vol. 34, no. 1, 1991, pages 439-447, XP002969016 ISSN: 0022-2623
- VINCENT M ET AL: "SYNTHESIS AND ACE INHIBITORY ACTIVITY OF THE STEREOISOMERS OF PERINDOPRIL (S 9490) AND PERINDOPRILATE (S 9780)" DRUG DESIGN AND DISCOVERY, HARWOOD ACADEMIC PUBLISHERS GMBH, XX, vol. 9, no. 1, 1992, pages 11-28, XP000885876 ISSN: 1055-9612

## Description

La présente invention concerne un procédé de synthèse de la N-[(S)-1-carbéthoxybutyl]-(S)-alanine, et son application à la synthèse du perindopril et de ses sels pharmaceutiquement acceptables.

Plus spécifiquement, la présente invention concerne un nouveau procédé de synthèse du composé de formule (I) : 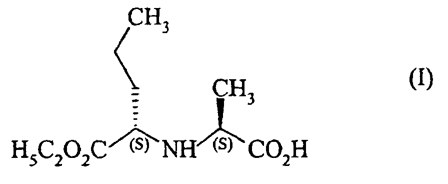 ainsi que ses sels d'addition à un acide ou à une base minéral(e) ou organique.

Le composé de formule (I) obtenu selon le procédé de l'invention est utile dans la synthèse du perindopril de formule (II) : 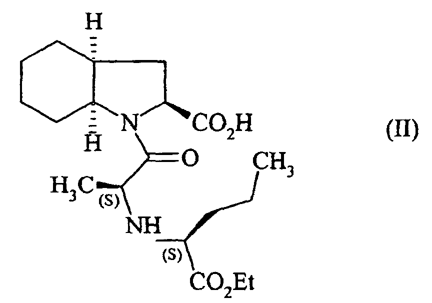 ainsi que dans celle de ses sels pharmaceutiquement acceptables.

Le perindopril, ainsi que ses sels, possèdent des propriétés pharmacologiques intéressantes. Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.

Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant.

Quelques méthodes de préparation du composé de formule (I) sont déjà connues.
- Le journal Tet. Lett. 1982, 23 (16), 1677-80 décrit l'obtention du composé de formule (I) par réaction dans l'éthanol du 2-oxovalérate d'éthyle avec l'ester tert-butylique de l'alanine en présence de cyanoborohydrure de sodium.
- Le brevet EP 0 309 324 décrit l'accès au composé de formule (I) par réaction dans le diméthylformamide de l'ester benzylique de l'alanine avec l'α-bromovalérate d'éthyle en présence de triéthylamine.
- Les brevets EP 0 308 340 et EP 0 308 341 décrivent l'accès au composé de formule (I) par réaction dans l'eau du chlorhydrate de norvalinate d'éthyle avec l'acide pyruvique en présence d'hydrogène, de charbon palladié et de soude.

La demanderesse a présentement mis au point un nouveau procédé de synthèse du composé de formule (I).

Plus spécifiquement, la présente invention concerne un procédé de synthèse du composé de formule (I), caractérisé en ce que l'on met en réaction le dérivé de l'alanine de formule (III) : 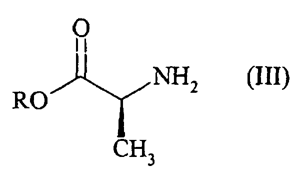 dans laquelle R représente un groupement alkyle (C₁-C₆) linéaire ou ramifié ou benzyle,
- soit avec le glyoxylate d'éthyle de formule (IVa) : 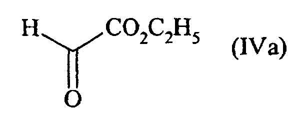
- soit avec le dérivé de formule (IVb) : 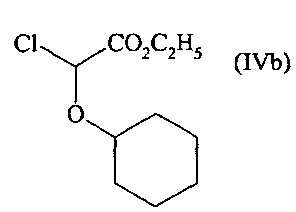
pour conduire à l'imine de formule (V) : 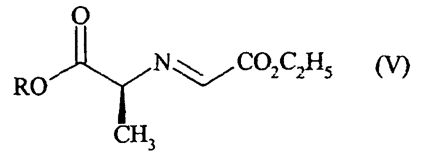 dans laquelle R est tel que défini précédemment,
que l'on met en réaction avec le composé de formule (VI) : 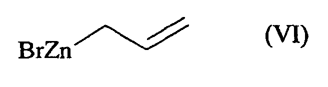 pour conduire après isolement au composé de formule (VII) : 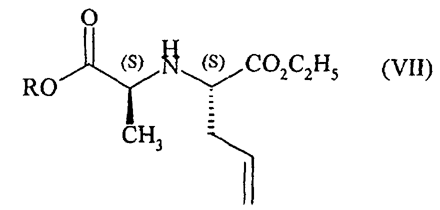 dans laquelle R est tel que défini précédemment,
que l'on soumet à une hydrogénation catalytique et à une transformation de la fonction CO₂R en fonction acide, pour conduire au composé de formule (I).

Les groupements R préférés sont les groupements tert-butyle et benzyle.

Dans le procédé de la présente invention, l'imine de formule (V) est préférentiellement obtenue par réaction du composé de formule (III) avec le dérivé de formule (IVb).

L'imine de formule (V) peut être isolée avant sa réaction avec le composé de formule (VI) ou engagée sans isolement, sous forme de solution.

Les composés de formule (V) et (VII) sont des produits nouveaux, utiles comme intermédiaires de synthèse dans l'industrie chimique ou pharmaceutique, notamment dans la synthèse du perindopril, et font à ce titre partie intégrante de la présente invention.

Le composé de formule (VI) est obtenu à partir de bromure d'allyle et de zinc en poudre.

### Exemple : N-[(S)-Carbéthoxy-1-butyl]-(S)-alanine, chlorhydrate

### Stade A : (2S)-2-{[(E)-2-Ethoxy-2-oxoéthylidène]amino}propanoate de tert-butyle :

A une solution de 9,1 g de chlorhydrate de (S)-alaninate de tert-butyle et de 18,5 ml de triéthylamine dans 35 ml de diméthylformamide sont ajoutés goutte à goutte 11 g d'α-chloro α-cyclohexyloxyacétate d'éthyle à 15°C, puis le mélange réactionnel est amené à température ambiante et agité 1h30.
La solution de (2S)-2-{[(E)-2-éthoxy-2-oxoéthylidène]-amino}-propanoate de tert-butyle ainsi obtenue est engagée telle quelle dans l'étape suivante.

### Spectrométrie de masse (sur produit isolé):

m/e (EI) : 229 (M),5 ; 173 (M-56), 16 ; 128 (M-CO₂tBu), 42; 100 (M-CH(CO₂tBu)CH₃), 100; 57 (tBu), 49.

### Stade B : (2S)-2-{[(1S)-1-Allyl-2-éthoxy-2-oxoéthyl]amino}propanoate de tert-butyle

A la solution obtenue dans le stade précédent est ajoutée au goutte à goutte une solution de bromure d'allylzinc, préparée au préalable par réaction de 13 ml de bromure d'allyle et 11,7 g de zinc en poudre dans le tétrahydrofurane. Après 24h d'agitation à température ambiante, 700 ml d'une solution aqueuse saturée de chlorure d'ammonium sont ajoutés. La phase organique est extraite à l'éther puis séchée, filtrée et concentrée, puis le résidu est repris dans l'éther et traité par 100 ml d'une solution aqueuse saturée en sulfure de sodium. Après 2h d'agitation, les deux phases sont séparées puis la phase organique est séchée, filtrée et évaporée, pour conduire au produit du titre.

### Spectrométrie de masse :

m/e (EI) : 230 (M-allyl),4 ; 170 (M-CO₂tBu), 100 ; 57 (tBu), 34 ; 41 (allyl), 38.

### Stade C : (2S)-2-{[(1S)-2-Tert-butyloxy-1-méthyl-2-oxoéthyl]amino}pentanoate d'éthyle :

Dans un hydrogénateur, placer 20 g du composé obtenu dans le stade précédent en solution dans l'éthanol, puis 0,5 g de Pd/C à 10 %. Hydrogéner sous pression normale et température ambiante, jusqu'à absorption de la quantité théorique d'hydrogène.
Eliminer le catalyseur par filtration, puis isoler le (2S)-2-{[(1S)-2-tert-butyloxy-1-méthyl-2-oxoéthyl]amino}pentanoate d'éthyle par mise à sec.

### Stade D : N-[(S)-Carbéthoxy-1-butyl]-(S)-alanine, chlorhydrate :

Le composé obtenu au stade précédent est déprotégé par réaction à température ambiante dans le dichlorométhane contenant 2% de tétrahydrofurane saturé en acide chlorhydrique, pour conduire après isolement au chlorhydrate de la N-[(S)-carbéthoxy-1-butyl]-(S)-alanine avec un rendement quantitatif.

## Revendications

1. Procédé de synthèse du composé de formule (I) : 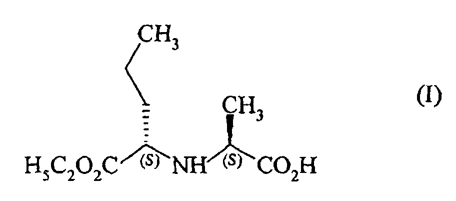 **caractérisé en ce que** l'on met en réaction le dérivé de l'alanine de formule (III) : 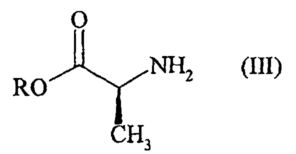 dans laquelle R représente un groupement alkyle (C₁-C₆) linéaire ou ramifié ou benzyle,
• soit avec le glyoxylate d'éthyle de formule (IVa) : 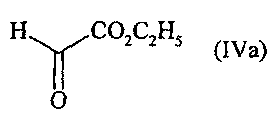
• soit avec le dérivé de formule (IVb) : 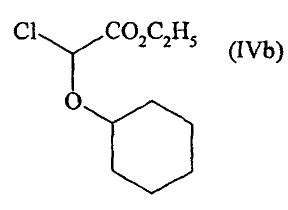 pour conduire à l'imine de formule (V) : 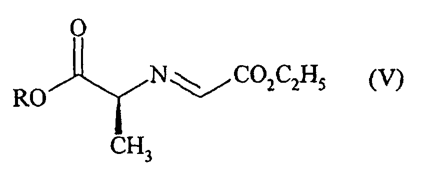 dans laquelle R est tel que défini précédemment,
que l'on met en réaction avec le composé de formule (VI) : 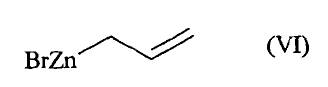 pour conduire après isolement au composé de formule (VII) : 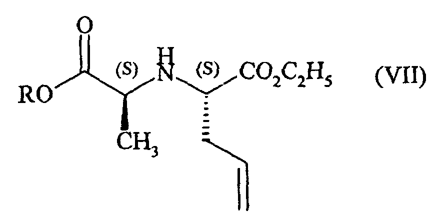 dans laquelle R est tel que défini précédemment,
que l'on soumet à une hydrogénation catalytique et à une transformation de la fonction CO₂R en fonction acide, pour conduire au composé de formule (I).

2. Procédé selon la revendication 1, **caractérisé en ce que** R représente un groupement benzyle ou tert-butyle.

3. Procédé selon l'une quelconque des revendications 1 ou 2, **caractérisé en ce que** l'imine de formule (V) est obtenue par réaction du composé de formule (III) avec le dérivé de formule (IVb).

4. Composé de formule (V) : 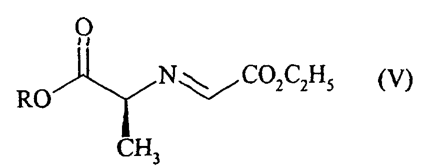 dans laquelle R représente un groupement alkyle (C₁-C₆) linéaire ou ramifié ou benzyle.

5. Composé de formule (VII) : 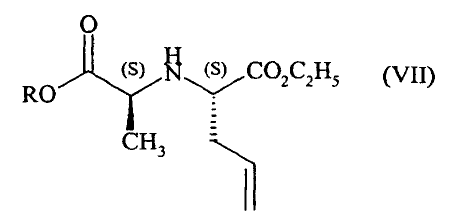 dans laquelle R représente un groupement alkyle (C₁-C₆) linéaire ou ramifié ou benzyle.

6. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur Synthese der Verbindung der Formel (I): 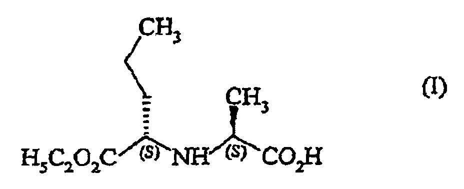 **dadurch gekennzeichnet, daß** man das Alanin-Derivat der Formel (III): 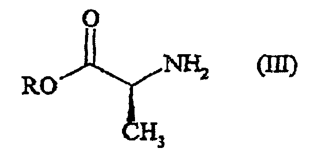 in der R eine geradkettige oder verzweigte (C₁-C₆)-Alkylgruppe oder eine Benzylgruppe bedeutet,
• entweder mit Glyoxylsäureethylester der Formel (IVa): 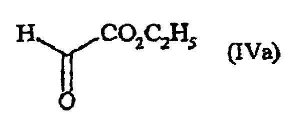
• oder mit dem Derivat der Formel (IVb): 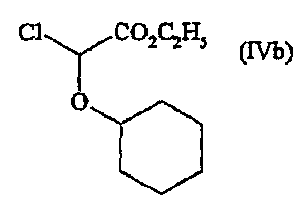
umsetzt zur Bildung des Imins der Formel (V): 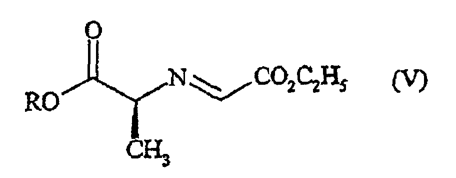 in der R die oben angegebenen Bedeutungen besitzt,
welches man mit der Verbindung der Formel (VI): 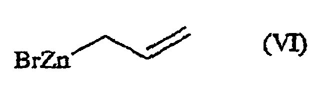 umsetzt, so daß man nach der Isolierung die Verbindung der Formel (VII) erhält: 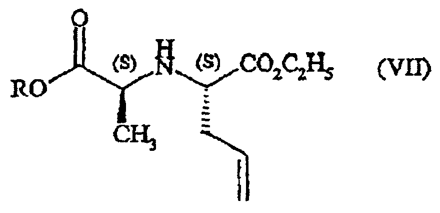 in der R die oben angegebenen Bedeutungen besitzt,
welche man einer katalytischen Hydrierung und einer Umwandlung der CO₂R-Funktion in eine Säurefunktion unterwirft zur Bildung der Verbindung der Formel (I).

2. Verfahren nach Anspruch 1, **dadurch gekennzeichnet, daß** R eine Benzyl- oder tert.-Butyl-Gruppe bedeutet.

3. Verfahren nach einem der Ansprüche 1 oder 2, **dadurch gekennzeichnet, daß** man das Imin der Formel (V) durch Umsetzen der Verbindung der Formel (III) mit dem Derivat der Formel (IVb) erhält.

4. Verbindung der Formel (V): 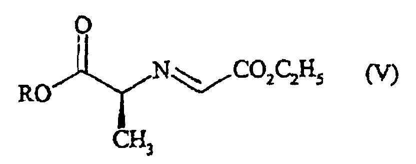 in der R eine geradkettige oder verzweigte (C₁-C₆)-Alkylgruppe oder eine Benzylgruppe bedeutet.

5. Verbindung der Formel (VII): 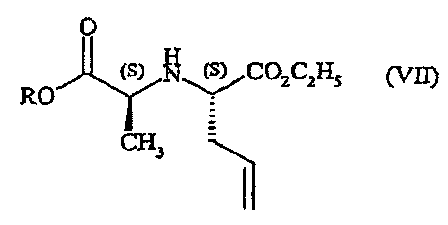 in der R eine geradkettige oder verzweigte (C₁-C₆)-Alkylgruppe oder eine Benzylgruppe bedeutet.

6. Verfahren zur Synthese von Perindopril oder von seinen pharmazeutisch annehmbaren Salzen, ausgehend von einer Verbindung der Formel (I), **dadurch gekennzeichnet, daß** man die Verbindung der Formel (I) nach dem Verfahren gemäß Anspruch 1 herstellt.

## Claims

1. Process for the synthesis of the compound of formula (1) : 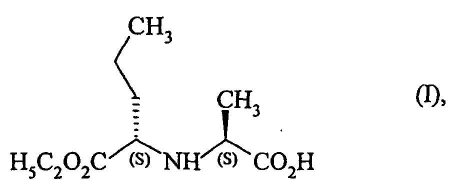 **characterised in that** the alanine compound of formula (III) : 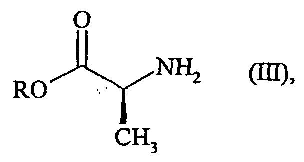 wherein R represents a linear or branched (C₁-C₆)alkyl or benzyl group, is reacted
• either with ethyl glyoxylate of formula (IVa) : 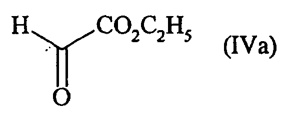
• or with the compound of formula (IVb) : 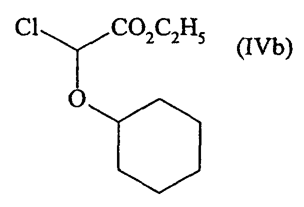 to yield the imine of formula (V) : 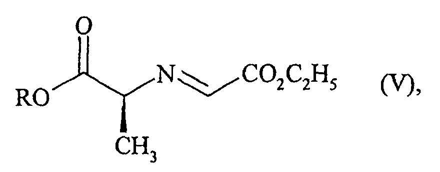 wherein R is as defined hereinbefore,
which is reacted with the compound of formula (VI) : 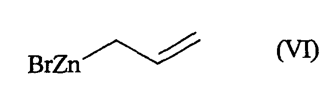 to yield, after isolation, the compound of formula (VII) : 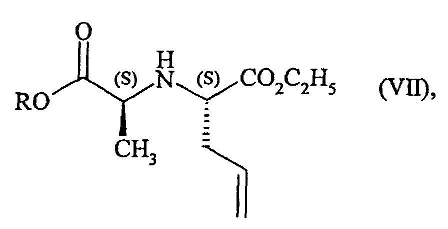 wherein R is as defined hereinbefore,
which is subjected to catalytic hydrogenation and to conversion of the CO₂R function into an acid function to yield the compound of formula (I).

2. Process according to claim 1, **characterised in that** R represents a benzyl or tert-butyl group.

3. Process according to either claim 1 or claim 2, **characterised in that** the imine of formula (V) is obtained by reaction of the compound of formula (III) with the compound of formula (IVb).

4. Compound of formula (V) : 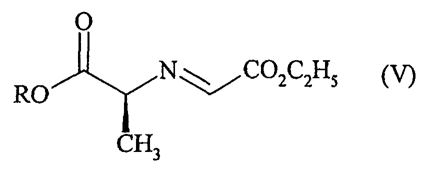 wherein R represents a linear or branched (C₁-C₆)alkyl or benzyl group.

5. Compound of formula (VII) : 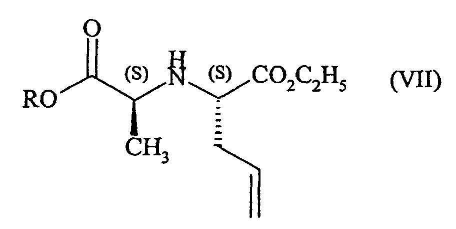 wherein R represents a linear or branched (C₁-C₆)alkyl or benzyl group.

6. Process for the synthesis of perindopril or pharmaceutically acceptable salts thereof starting from a compound of formula (I), **characterised in that** the said compound of formula (I) is obtained according to the process of claim 1.
